# EUROPEAN PATENT SPECIFICATION

(11) **EP 1 638 548 B1**
(45) Date of publication and mention of the grant of the patent: **29.09.2010**
(21) Application number: 04737850.0
(22) Date of filing: 18.06.2004
(51) Int. Cl.: A61K 31/222, A61K 31/351, A61P 9/06

(54) **PREVENTING ATRIAL FIBRILLATION (AF) WITH THE USE OF STATIN DRUGS**
VERMEIDUNG VON VORHOFFLIMMERN (AF) MIT DER VERWENDUNG VON STATIN
PREVENTION DE LA FIBRILLATION AURICULAIRE PAR DES MEDICAMENTS A BASE DE STATINES

(30) Priority: 18.06.2003 US 479147 P
(43) Date of publication of application: 29.03.2006
(73) Proprietor: Institut de Cardiologie de Montreal / Montréal Heart Institute, Montreal, Quebec H1T 1C8 (CA)
(72) Inventor: NATTEL, Stanley, Côte St-Luc, Québec H4V 2X6 (CA)
(74) Representative: Turner, Craig Robert
(86) International application number: PCT/CA2004/000911
(87) International publication number: WO 2004/110427

(56) References cited:
- US-A- 4 578 400
- US-B1- 6 218 403
- KUMAGAI KOICHIRO ET AL: "The HMG-CoA reductase inhibitor atorvastatin prevents atrial fibrillation by inhibiting inflammation in the canine sterile pericarditis model." JOURNAL OF THE AMERICAN COLLEGE OF CARDIOLOGY, vol. 41, no. 6 Supplement A, 19 March 2003 (2003-03-19), page 90A, XP008036415 & 52ND ANNUAL SCIENTIFIC SESSION OF THE AMERICAN COLLEGE OF CARDIOLOGY; CHICAGO, IL, USA; MARCH 30-APRIL 02, 2003 ISSN: 0735-1097
- RIDKER: "plasma homocysteine concentration" CIRCULATION, 2002, pages 1776-9, XP008036494
- CHUNG: "c-reactive protein elevation..." CIRCULATION, 2001, pages 2886-91, XP008036493
- WEST M ET AL: "Risk factors for stroke and the effect of cholesterol-lowering therapy with pravastatin" JOURNAL OF HYPERTENSION, vol. 18, no. Suppl. 4, 2000, page S17, XP008036422 & 18TH SCIENTIFIC MEETING OF THE INTERNATIONAL SOCIETY OF HYPERTENSION; CHICAGO, ILLINOIS, USA; AUGUST 20-24, 2000 ISSN: 0263-6352
- CHEN, JIANGUANG ET AL: "Pravastatin prevents arrhythmias induced by coronary artery ischemia/reperfusion in anesthetized normocholesterolemic rats" JOURNAL OF PHARMACOLOGICAL SCIENCES (TOKYO, JAPAN) , 93(1), 87-94 CODEN: JPSTGJ; ISSN: 1347-8613, 2003, XP008036429
- AKAHANE TAKEMI ET AL: "Atrial fibrillation induced by simvastatin treatment in a 61-year-old man." HEART AND VESSELS, vol. 18, no. 3, July 2003 (2003-07), pages 157-159, XP008036410 ISSN: 0910-8327
- SHIROSHITA-TAKESHITA AKIKO ET AL: "Differential efficacy of drugs with antioxidant properties on atrial fibrillation promotion by atrial tachycardia remodeling in dogs." CIRCULATION, vol. 108, no. 17 Supplement, 28 October 2003 (2003-10-28), pages IV-148, XP008036418 & AMERICAN HEART ASSOCIATION SCIENTIFIC SESSIONS 2003; ORLANDO, FL, USA; NOVEMBER 09-12, 2003 ISSN: 0009-7322
- SIU C-W ET AL: "Prevention of atrial fibrillation recurrence by statin therapy in patients with lone atrial fibrillation after successful cardioversion" AMERICAN JOURNAL OF CARDIOLOGY 01 DEC 2003 UNITED STATES, vol. 92, no. 11, 1 December 2003 (2003-12-01), pages 1343-1345, XP008036412 ISSN: 0002-9149
- KUMAGAI K ET AL: "The HMG-CoA reductase inhibitor atorvastatin prevents atrial fibrillation by inhibiting inflammation in a canine sterile pericarditis model" CARDIOVASCULAR RESEARCH 01 APR 2004 NETHERLANDS, vol. 62, no. 1, 1 April 2004 (2004-04-01), pages 105-111, XP008036414 ISSN: 0008-6363
- DERNELIS: "relationship between c reactive protein concentrations..." EUR. HEART J., 2004, pages 1100-7, XP008036504

## Description

### FIELD OF THE INVENTION

The present invention relates to the attenuation of atrial fibrillation (AF) promotion by atrial tachycardia (AT). Specifically, the invention concerns the use of HMG-CoA reductase inhibitors namely statin drugs, such as simvastatin (Zocor®), lovastatin (Mevacor®, Altocor®) and pravastatin (Pravachol®), to prevent and lessen AF promotion by atrial tachycardia.

### BACKGROUND OF THE INVENTION

Atrial fibrillation (AF) occurs when the electrical impulses in the atria degenerate from their usual organized pattern into a rapid chaotic pattern. This disruption results in an irregular and often rapid heartbeat that is classically described as "irregularly irregular" and is due to the unpredictable conduction of these disordered impulses across the atrioventricular (AV) node.

AF may be classified on the basis of the frequency of episodes and the ability of an episode to convert back to sinus rhythm. One method of classification is outlined in guidelines published by the American College of Cardiology (ACC), the American Heart Association (AHA), and the European Society of Cardiology (ESC), with the collaboration of the North American Society of Pacing and Electrophysiology (NASPE). According to these guidelines, if a patient has two or more episodes, AF is considered to be *recurrent.* Recurrent AF may be paroxysmal or persistent. If the AF terminates spontaneously it is designated as *paroxysmal,* and if the AF is sustained it is designated as *persistent.* In the latter case, termination of the arrhythmia with electrical or pharmacologic cardioversion does not change its designation. Persistent AF may present either as the first manifestation of the arrhythmia or as the culmination of recurrent episodes of paroxysmal AF. The category of persistent AF also includes *permanent* AF, which refers to long-standing (generally > 1 year) AF for which cardioversion was not indicated or attempted.

AF is the most common sustained tachyarrhythmia encountered by clinicians. AF occurs in approximately 0.4% to 1.0% of the general population, and it affects more than 2 million people in the United States annually. Its prevalence increases with age, and up to 10% of the population older than 80 years has been diagnosed with AF at some point. With the projected growth of the elderly population the prevalence of AF will certainly increase.

AF may be associated with physiologic stresses such as surgical procedures, pulmonary embolism, chronic lung diseases, hyperthyroidism, and alcohol ingestion. Disease states commonly associated with AF include hypertension, valvular heart disease, congestive heart failure (CHF), coronary artery disease, Wolff-Parkinson-White (WPW) syndrome, pericarditis, and cardiomyopathy. When no identifiable risk factor for AF is present, the condition is classified as *lone* AF.

AF may have hemodynamic consequences. It may decrease the cardiac output by as much as 20%, increase pulmonary capillary wedge pressure, and increase atrial pressures. These effects are due to tachycardia, loss of atrial contribution to left ventricular (LV) filling, increased valvular regurgitation, and the irregular ventricular response. Some investigators have suggested that the irregularity of the R-R intervals contributes more to the hemodynamic changes than does the mere presence of tachycardia.

The clinical presentation of AF is quite variable. Generally the symptoms are attributable to the rapid ventricular response. However, even when the ventricular response is controlled, symptoms may occur from the loss of AV synchrony. This is particularly the case for patients with LV dysfunction. Some patients are completely asymptomatic, even those who have rapid heart rates.

But more often, patients report nonspecific symptoms such as fatigue, dyspnea, dizziness, and diaphoresis. Palpitations are a common feature. Occasionally, patients present with extreme manifestations of hemodynamic compromise, such as chest pain, pulmonary edema, or syncope. AF is present in 10% to 40% of patients with a new thromboembolic stroke.¹

AF is difficult to treat. Atrial tachyarrhythmias alter atrial electrophysiology in a way that promotes AF, and these alterations are believed to contribute to both the occurrence and persistence of the arrhythmia.²⁻⁵ Prevention of atrial tachycardia-induced remodeling is an attractive therapeutic approach,⁶ but to date the only drugs shown to prevent experimental remodeling due to several days or more of atrial tachycardia are mibefradil, which is no longer on the market, and amiodarone.⁹

There is therefore a need for a new pharmaceutical approach to prevent the consequences of atrial tachycardia remodeling.

### SUMMARY OF THE INVENTION

According to the present invention, therefore, there is provided the use of a therapeutically effective amount of a HMG - CoA reductase inhibitor, namely a statin drug such as simvastatin ( Zocor®) in the manufacture of a medicament for preventing or attenuating atrial fibrillation (AF) promotion by atrial tachycardia in a subject.

There is evidence for enhanced oxidative stress in atrial tissue samples from AF patients¹⁰ and for benefit from antioxidant vitamins in preventing atrial tachycardia remodeling.¹¹ In addition, there is evidence for a role of inflammation in AF.^{12,13} Statins have both anti-inflammatory and antioxidant properties.^{14,15}

A study was designed to assess the effects of simvastatin on atrial remodeling caused by one week of atrial tachycardia. As comparator agents, vitamin C and combined vitamins C and E therapy were used, since these also have some antioxidant properties and vitamin C has shown some value in preventing atrial tachycardia remodeling.¹¹

Simvastatin is known to reduce oxidant stress and inflammation, processes believed to play a role in AF. The effects of simvastatin with antioxidant vitamin C and vitamins C plus E on atrial remodeling in dogs caused by atrial tachypacing (400 bpm) were compared. Serial closed-chest electrophysiological studies were performed in each dog at baseline and 2, 4, and 7 days after tachypacing-onset. Atrioventricular block was performed to control ventricular rate. Mean duration of induced AF was increased from 42±18 seconds to 1079±341 seconds at terminal open-chest study after tachypacing alone (*P*<0.01), and atrial effective refractory period (ERP) at a cycle length of 300 ms was decreased from 117±5 to 76±6 ms (*P*<0.01). Tachypacing-induced ERP shortening and AF promotion were unaffected by vitamin C or vitamins C and E; however, simvastatin suppressed tachypacing-remodeling effects significantly, with AF duration and ERP averaging 41±15 seconds and 103±4 ms respectively after tachypacing with simvastatin therapy. Tachypacing downregulated L-type Ca²⁺-channel α-subunit expression (Western blot), an effect that was unaltered by antioxidant vitamins but greatly attenuated by simvastatin.

One week of tachypacing abbreviated atrial refractoriness and increased AF duration, an effect not altered by vitamin C or vitamin C plus E. Simvastatin attenuated atrial ERP abbreviation and AF promotion caused by atrial tachypacing, and prevented tachypacing-induced downregulation of Caᵥ1.2 protein. Simvastatin therefore prevented atrial tachycardia-induced remodeling.

Other objects, advantages and features of the present invention will become more apparent upon reading of the following description of preferred embodiments thereof, given by way of example only with reference to the accompanying drawings.

### BRIEF DESCRIPTION OF THE DRAWINGS

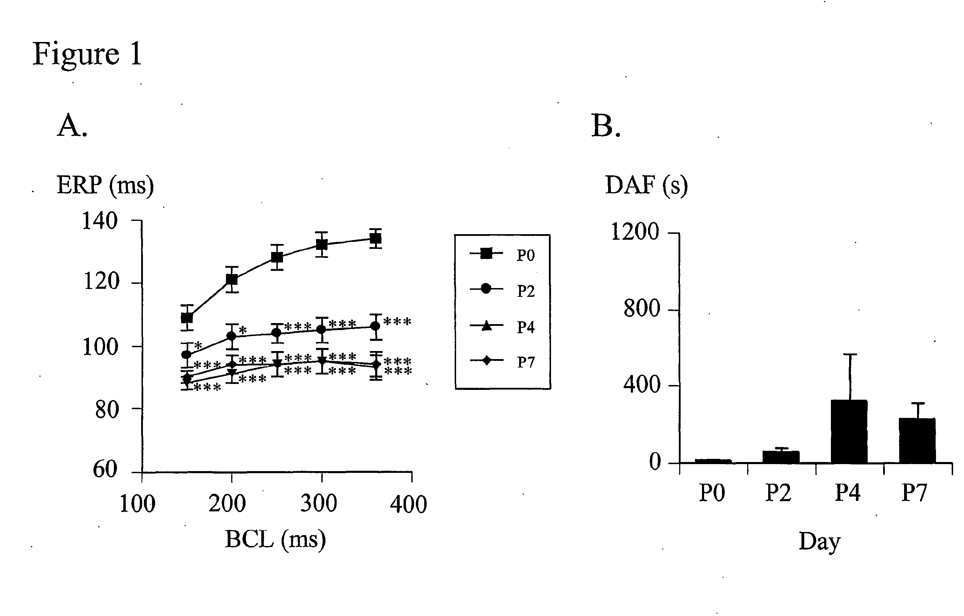

**Figure 1****:** The effect of atrial tachypacing on electrophysiological parameters during closed-chest study in ATP-only dogs. **A,** ERP as a function of basic cycle length (BCL) at baseline (day 0, P0) and after 2 (P2), 4 (P4) and 7 (P7) days of ATP. **B,** Mean duration of induced AF (DAF) as a function of tachypacing duration. **P*<0.05, ****P*<0.001 versus P0.

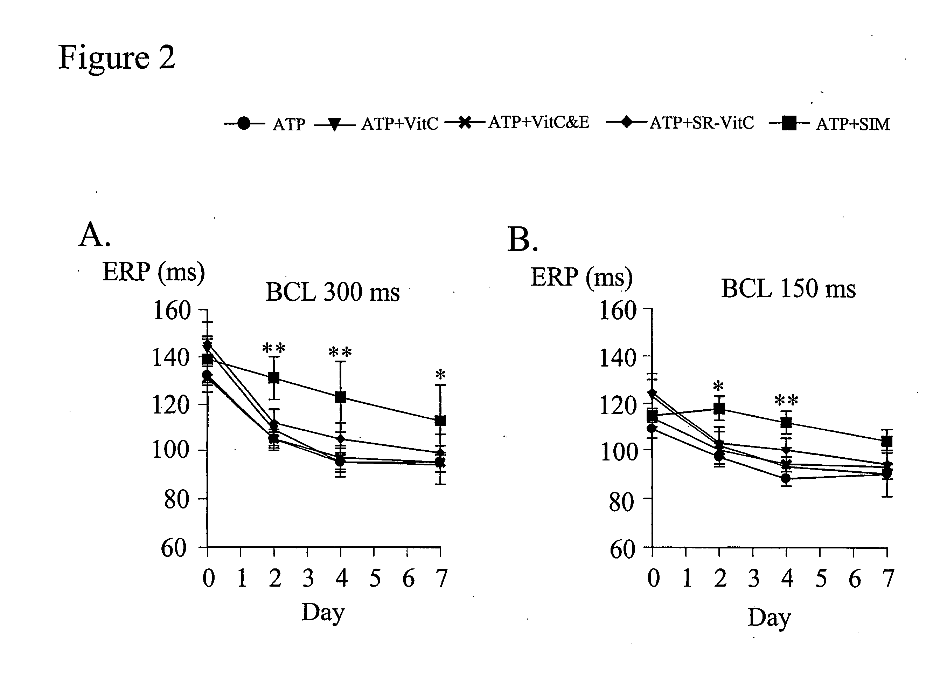

**Figure 2****:** Time-dependent ERP changes as measured during serial closed chest study at basic cycle lengths (BCLs) of 300 **(A)** and 150 **(B)** ms, after atrial tachypacing for the durations indicated. **P*<0.05, ***P*<0.01 versus ATP-only. ATP=ATP-only; ATP+VitC, ATP+VitC&E, ATP+SR-VitC, ATP+SIM=atrial tachypacing in presence of vitamin C, vitamins C and E, sustained-release vitamin C and simvastatin, respectively.

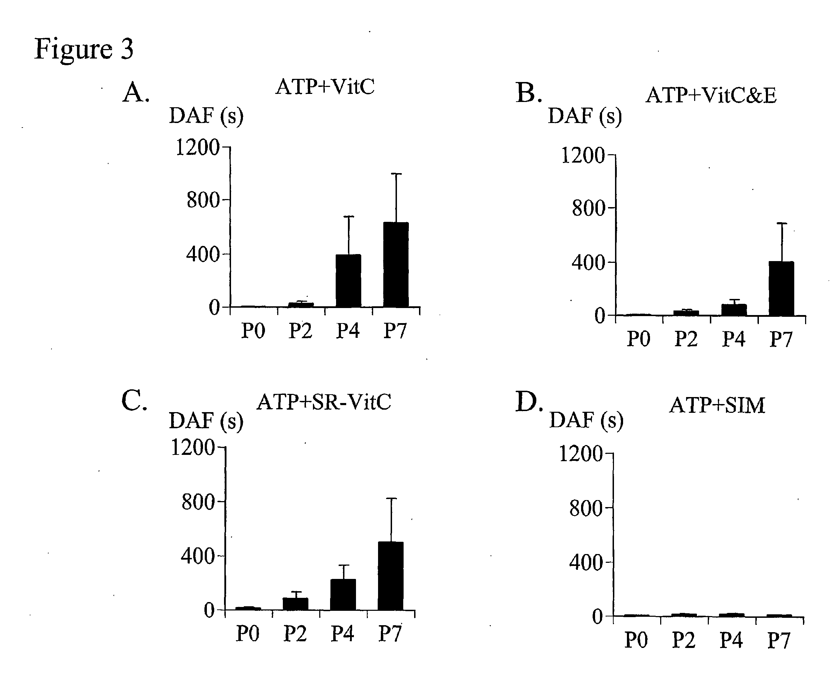

**Figure 3****:** Mean±SEM AF duration (DAF) during 7-day atrial tachypacing and treatment with: vitamin C **(panel A);** vitamins C and E **(panel B);** sustained-release vitamin C **(panel C);** simvastatin **(panel D).** P0, P2, P4, P7=pacing for 0, 2, 4 and 7 days, respectively.

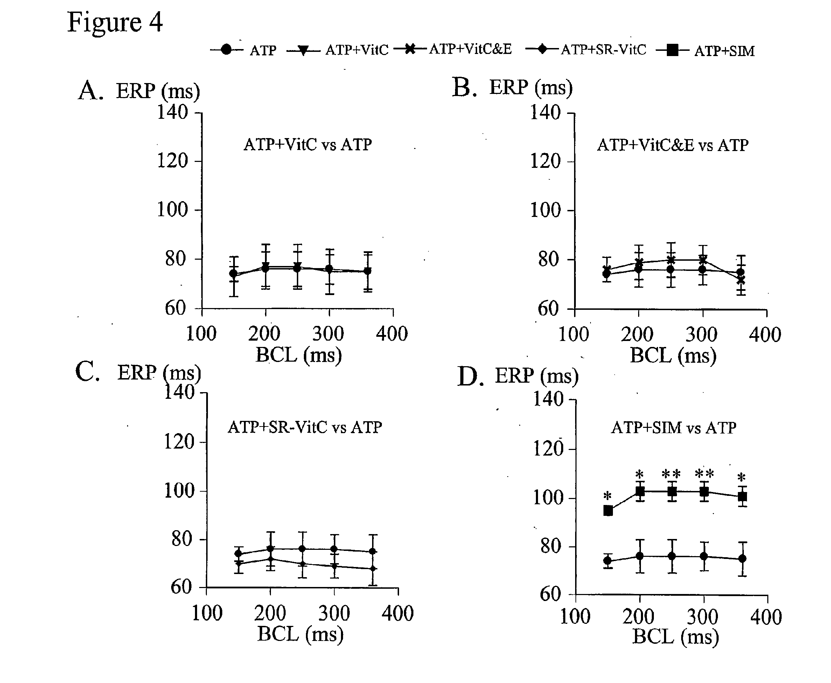

**Figure 4****:** Mean±SEM ERP values in RA appendage during the final open-chest study. No significant differences were observed between ATP-only dogs and vitamin C-treated **(A),** vitamin C and E treated **(B),** or sustained-release vitamin C-treated dogs **(C).** ERP values were significantly greater in simvastatin-treated dogs than in ATP-only dogs **(D).** **P*<0.05, ***P*<0.01 versus ATP-only. Abbreviations as in Figure 2.

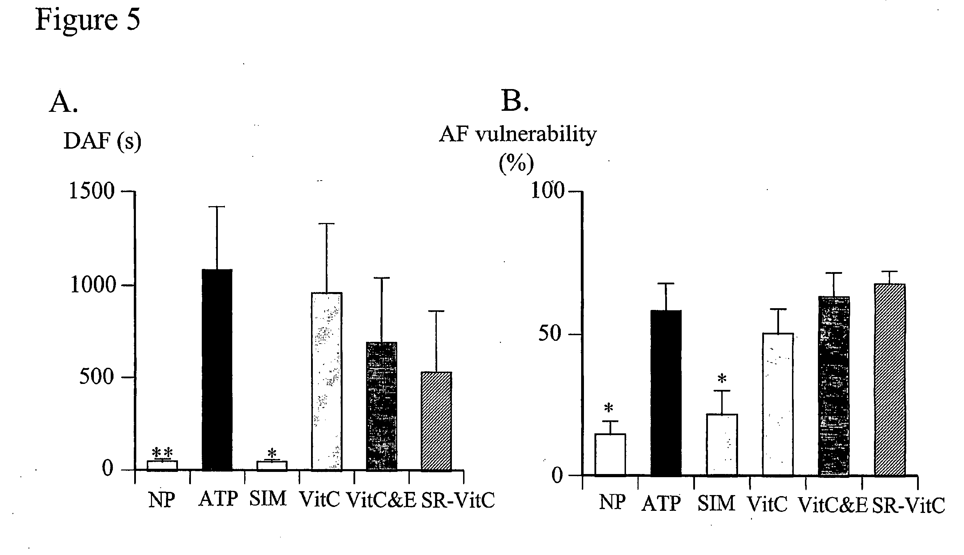

**Figure 5****:** AF promotion as measured during final open-chest study. **A,** mean±SEM duration of AF (DAF). **B,** AF vulnerability, as percentage of sites at which AF could be induced by single premature extrastimuli. **P*<0.05, ***P*<0.01 versus ATP-only. NP=non-paced controls; ATP=ATP-only; SIM, VitC, VitC&E, SR-VitC=atrial tachypaced dogs treated with simvastatin, vitamin C, vitamins C and E, and sustained-release vitamin C, respectively.

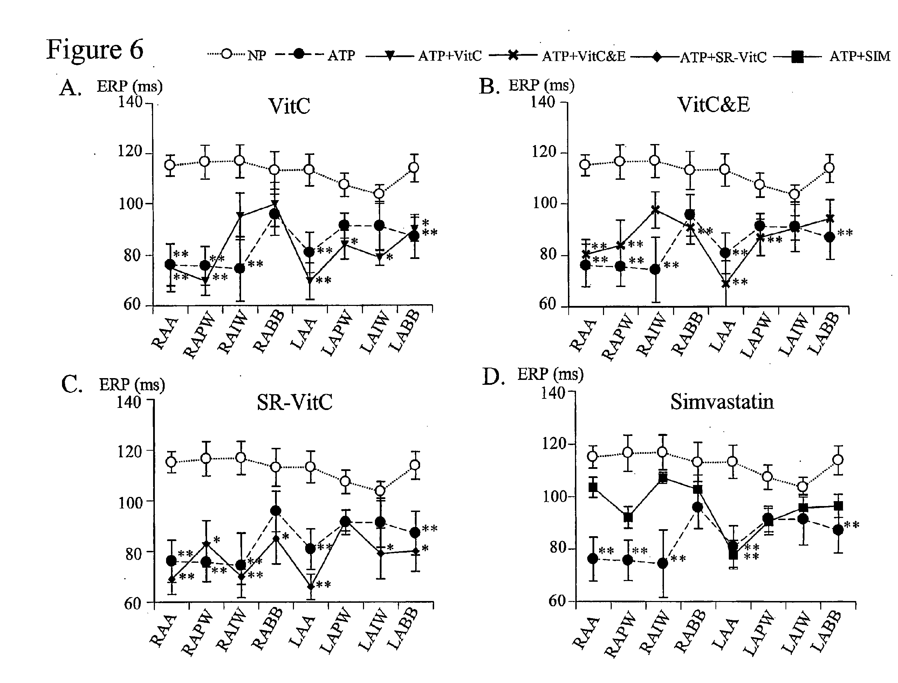

**Figure 6****:** ERPs in different atrial regions at BCL 300 ms at final open-chest study. In each panel, results from non-paced dogs are shown by dotted lines (·····) and results from ATP-only dogs by dashed lines (-------). These are compared to results in ATP dogs treated with vitamin C **(A),** vitamins C and E **(B),** sustained-release vitamin C **(C)** and simvastatin **(D).** **P*<0.05, ***P*<0.01 versus NP. RAA, RAPW, RAIW, RABB, LAA, LAPW, LAIW, LABB=RA and LA appendage, posterior wall, inferior wall, Bachmann's bundle, respectively.

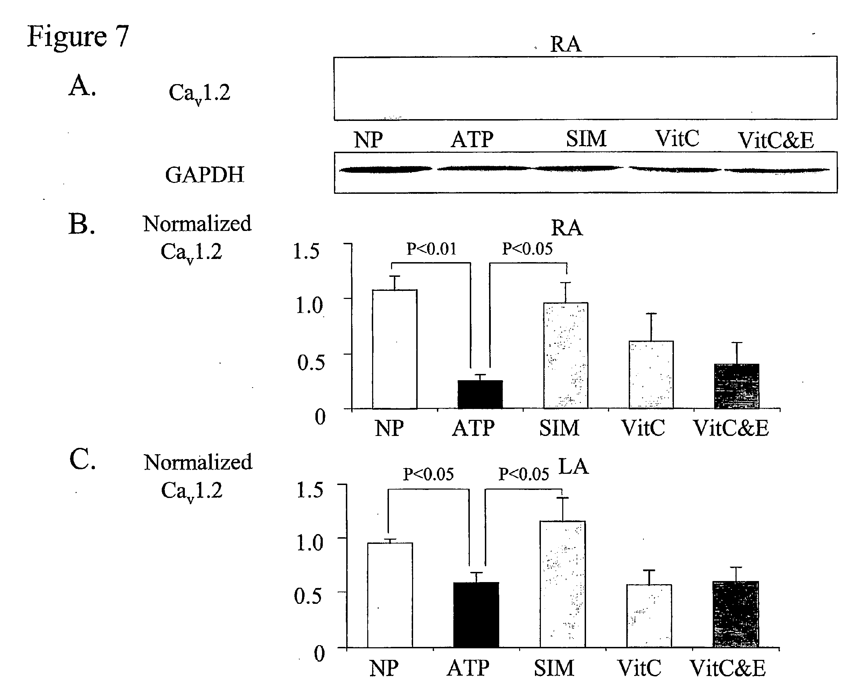

**Figure 7****:** Expression of L-type Ca²⁺-channel α-subunit (Caᵥ1.2) protein. **A,** Representative results from single gel. **B-C**, mean±SEM Caᵥ1.2 protein band intensities (normalized to GAPDH and control band in each gel) in RA **(B)** and LA **(C)** appendages. Abbreviations as in Figure 5.

### DESCRIPTION OF THE PREFERRED EMBODIMENTS

**Definitions:** Unless otherwise specified, the terms used herein have the meanings that would be understand by those of skill in the art. For convenience, the following recurring terms have been defined.

**"ATP":** Atrial tachypacing.

**"Atrial fibrillation":** Atrial fibrillation (often termed "AF") is a heart rhythm disorder (arrhythmia). It usually involves a rapid heart rate, in which the upper heart chambers (atria) are stimulated to contract in a very disorganized and abnormal manner.

**"Atrial fibrillation promotion":** A process that makes AF easier to initiate or maintain.

**"Atrial tachyarrhythmia":** A too-rapid, irregular rhythm in the heart's upper chambers that can impair a person's quality of life. If left untreated, atrial tachyarrhythmias can lead to a fivefold increase in the risk of stroke.

**"Atrial tachycardia":** A sustained, irregular heart rhythm that occurs in the upper chamber of the heart and causes it to beat too rapidly.

**"Atrial tachycardia-induced remodeling",** or **"atrial tachycardia remodeling":** These terms define the changes of atrial electrophysiologic properties taking place in atrial myocytes during atrial fibrillation and/or following periods of sustained atrial fibrillation (AF). This is also called "electrophysiological remodeling".

**"BCL":** Basic cycle length.

**"CRP":** C-reactive protein (CRP), a marker for inflammation, is analyzed as a predictor of cardiovascular disease. CRP is a pentameric globulin with mobility near the gamma zone. It is an acute phase reactant which rises rapidly, but nonspecifically in response to tissue injury and inflammation. It is particularly useful in detecting occult infections, acute appendicitis, particularly in leukemia and in postoperative patients. In uncomplicated postoperative recovery, CRP peaks on the 3rd post-op day, and returns to pre-op levels by day 7. It may also be helpful in evaluating extension or reinfarction after myocardial infarction, and in following response to therapy in rheumatic disorders.

**"DAF":** Duration of induced AF.

**"EPS":** Electrophysiological study.

**"Extracardiac action":** Action on organs other than the heart.

**"ERP":** effective refractory period.

**"LA":** Left atrium.

**"HMG-Co A reductase inhibitor":** 3-Hydroxy-3-Methyl-Glutaryl Coenzyme A reductase inhibitors ("statins") are compounds that act by blocking an enzyme that is needed by the body to make cholesterol. Sometimes referred to antihyperlipidemic drugs, they thus help to lower cholesterol in the body. Members include the following medicaments: atorvastatin (Lipitor®), cerivastatin (Baycol®), fluvastatin (Lescol®), lovastatin (Mevacor®, Altocor®), pravastatin (Pravachol®), simvastatin (Zocor®), epistatin, eptastatin, mevinolin, and synvinolin. They have a common mechanism of action and are thought to behave in a biologically similar fashion.

**"RA":** Right atrium.

**"R-R interval":** The time between two consecutive heartbeats measured by the distance from one ECG QRS complex to the next QRS complex. Note that the average R-R interval in seconds is obtained by dividing 60 seconds by the heart rate measured. In normal individuals, the R-R interval is somewhat variable.

### Experimental

### Animal Model

Thirty-nine mongrel dogs (body weight 20 to 37 kg) were anesthetized with ketamine (5.3 mg/kg IV), diazepam (0.25 mg/kg IV), and halothane (1.5%). Unipolar leads were inserted through jugular veins into the right ventricular (RV) apex and right atrial (RA) appendage under fluoroscopic guidance and connected to pacemakers (Medtronic) in subcutaneous pockets in the neck. A bipolar electrode was also inserted into the RA for atrial stimulation and recording during serial electrophysiological study (EPS). AV block was created by radiofrequency catheter ablation to control ventricular response during atrial tachypacing (ATP) and the RV pacemaker was programmed to pace at 80 bpm. After 24 hours for recovery, a baseline closed-chest EPS was performed under anesthesia with ketamine, diazepam, and isoflurane, and then ATP (400 bpm) was initiated. Closed-chest EPS was repeated at 2, 4 and 7 days of ATP and a final open-chest EPS was performed on day 8 under anesthesia with morphine and α-chloralose.

### Groups

Results in 7 atrial tachypaced dogs without any treatment (ATP-only group) and 9 non-paced control dogs were each compared with those of dogs subjected to ATP during oral treatment with: 1) simvastatin, 80 mg/day (n=6), beginning 3 days prior to ATP onset; 2) vitamin C, 500 mg twice daily (n=6); and 3) combined vitamin C, 500 mg and vitamin E, 200 IU twice daily (n=6), beginning 1 day prior to ATP onset and continued throughout the study period. In addition, because no clear effect of vitamin C was observed at this dose, we studied an additional group of 5 dogs receiving sustained-release vitamin C 1.5 g daily in divided doses beginning 1 day before ATP.

### Study Protocol

On each closed-chest EPS day, dogs were anesthetized with ketamine (5.3 mg/kg IV), diazepam (0.25 mg/kg IV), and isoflurane (1.5%), and ventilated mechanically. The atrial pacemaker was then deactivated and the RA appendage effective refractory period (ERP) was measured at basic cycle lengths (BCLs) of 150, 200, 250, 300, and 360 ms. ERP was measured with 10 basic stimuli (S1) at various BCLs followed by a premature extrastimulus (S2) with 5 ms decrements. The longest S1-S2 failing to capture the atria defined the ERP. AF was induced with atrial burst pacing at 10 Hz and 4 times threshold current. Mean AF duration was calculated based on 10 inductions for AF <20 minutes and 5 inductions for AF lasting 20 to 30 minutes. AF lasting longer than 30 minutes was considered sustained and terminated by DC cardioversion. A 20-minute rest period was then allowed before continuing measurements. If sustained AF was induced twice during an experiment, no further AF induction was performed.

For open-chest EPS, dogs were anesthetized with morphine (2 mg/kg SC) and α-chloralose (120 mg/kg IV, followed by 29.25 mg/kg/h), and ventilated mechanically. Body temperature was maintained at 37°C, and a femoral artery and both femoral veins were cannulated for pressure monitoring and drug administration. A median sternotomy was performed, and bipolar electrodes were hooked to the RA and left atrial (LA) appendage for recording and stimulation. A programmable stimulator (Digital Cardiovascular Instruments) was used to deliver twice-threshold currents. Five silicon sheets containing 240 bipolar electrodes were sutured onto the atrial surfaces as previously described.⁷⁻⁹ Atrial ERP was measured over a range of BCLs in RA and LA appendages and at BCL 300 ms in 6 additional sites: RA posterior wall, RA inferior wall, RA Bachmann's bundle, LA posterior wall, LA inferior wall, and LA Bachmann's bundle. AF duration was assessed as described above and AF vulnerability was determined as the percentage of atrial sites at which AF could be induced by single extrastimuli.

Blood samples were collected on the final open-chest study day. Serum was removed following centrifugation (3000 rpm, 20 minutes) and stored at -80°C for subsequent C-reactive protein (CRP) analysis. CRP was measured with the Phase Range® canine CRP ELISA kit (Tri-delta Diagnostics Inc.).

At the end of open-chest studies, RA and LA tissue samples were fast-frozen in liquid nitrogen and stored at -80°C. To isolate proteins, tissues were homogenized in RIPA buffer with a protease inhibitor cocktail (5 µg/µL leupeptin, 5 µg/µL soybean trypsin inhibitor and 10 µg/mL benzamidine; Sigma) added to prevent protein degradation. The suspension was incubated on ice and then centrifuged (14000 g, 10 minutes, 4°C). The soluble fraction was stored at -80°C. Protein concentrations were measured by Bradford assay with bovine albumin as a standard. Proteins (200-µg samples) were denatured in Laemmli buffer, electrophoresed on 7.5% SDS-polyacrylamide gels and then transferred to polyvinylidene difluoride (PVDF) membranes overnight, blocked for 2 hours with 0.1% Tween-80-Tris-buffered saline (TTBS) at room temperature (RT) and then incubated with primary antibody (Alomone Labs, anti-cardiac Caᵥ1.2, 1:100) at 4°C overnight. After 3 washes, membranes were re-blocked in 1% nonfat dry milk in TTBS for 10 minutes and incubated with secondary antibody (Jackson Laboratories, goat anti-rabbit) for 90 minutes at RT. After 3 additional washes in TTBS, antibody detection was performed with Western Lightning™ Western Blot Chemiluminescence Reagent *Plus*. Band densities were quantified by densitometry (Quantity One software) standardized to GAPDH and normalized to the control sample on each gel.

### Data Analysis

Data are presented as mean±SEM. Multiple-group comparisons were obtained by ANOVA. A *t*-test with Bonferroni correction was used to evaluate differences between individual means. A two-tailed P<0.05 was considered statistically significant.

### Results

### General Properties

There were no significant differences among groups in mean body weight or in hemodynamic variables at final open-chest study (Table 1). Although CRP tended to be slightly higher at end-study in ATP-only dogs, CRP varied widely among dogs and there were no statistically-significant CRP differences among groups.

**TABLE 1: GENERAL PROPERTIES OF EACH GROUP AT OPEN-CHEST STUDY**

| | **NP** | **ATP alone** | **ATP+ VitC** | **ATP+ VitC&E** | **ATP+ SR-VitC** | **ATP+ SIM** | ***P*** |
|---|---|---|---|---|---|---|---|
| Body weight, kg | 30±1 | 30±0 | 32±2 | 29±1 | 35±2 | 29±2 | NS |
| | | | | | | | |
| HR, bpm | 158±6 | 165±9 | 145±9 | 150±8 | 144±9 | 169±6 | NS |
| Systolic BP, mm Hg | 104±7 | 108±16 | 107±8 | 109±9 | 104±6 | 131±4 | NS |
| Diastolic BP, mm Hg | 58±7 | 50±8 | 41±6 | 56±9 | 54±6 | 60±7 | NS |
| | | | | | | | |
| LVSP, mm Hg | 106±8 | 102±14 | 107±8 | 107±9 | 113±6 | 127±5 | NS |
| LVEDP, mm Hg | 6±1 | 6±1 | 6±1 | 5±1 | 4±1 | 9±1 | NS |
| LAP, mm Hg | 5±1 | 6±1 | 5±1 | 4±1 | 4±1 | 8±1 | NS |
| CRP, mg/L | 17±6 | 24±8 | 20±9 | 30±8 | N.A. | 14±5 | NS |

| | | | | | | | |
|---|---|---|---|---|---|---|---|
| NP indicates non-paced control group; ATP, atrial tachypacing-only group; ATP+VitC, ATP with vitamin C treatment; ATP+VitC&E, ATP with combined vitamin C and E treatment; ATP+SR-vitC, ATP with slow-release vitamin C; ATP+SIM, ATP with simvastatin treatment; HR, heart rate; BP, blood pressure; LVSP, left ventricular systolic pressure; LVEDP, left ventricular end-diastolic pressure; LAP, left atrial pressure; NA, not available. | | | | | | | |

### Effects of Interventions on Atrial Tachycardia-Induced Changes as Measured During Serial

### Closed-Chest Studies

Changes in ERP caused by 7 days of ATP in ATP-only dogs are shown in Figure 1. ERP decreased substantially within 2 days and reached steady-state changes at 4 days (Figure 1 A). AF duration increased substantially from 10±7 seconds prior to ATP to values averaging hundreds of seconds on days 4 and 7 of atrial tachycardia (Figure 1 B).

Figure 2 compares ERP changes as measured at cycle lengths of 300 (panel A) and 150 (panel B) ms in dogs subjected to ATP-only with dogs subjected to atrial tachycardia in the presence of each of the drug interventions. Under baseline conditions (day 0) there were no significant differences in ERP among groups. With the onset of atrial tachycardia, ERP decreased rapidly and to a similar extent in ATP-only dogs and in dogs subjected to atrial tachycardia in the presence of each of the antioxidant vitamin regimens. In simvastatin-treated dogs subjected to atrial tachycardia, the ERP changes were smaller and ERP values were larger than in ATP-only dogs for both cycle lengths.

Figure 3 shows the progression of mean AF duration in dogs subjected to atrial tachycardia in the presence of each of the interventions studied. With vitamin C (panel A), vitamins C and E (panel B) and sustained-release vitamin C (panel C), progressive increases in AF duration to means between -400 to 600 seconds occurred by day 7, not significantly different from ATP-only dogs. In contrast, atrial tachycardia-induced AF promotion was virtually abolished in simvastatin-treated dogs (panel D).

### Differences Among Groups in Results at Final Open-Chest Study

ERP values measured as a function of cycle length during the final open-chest study are illustrated in Figure 4. Dogs subjected to ATP without drug intervention had atrial ERPs averaging <80 ms at all basic cycle lengths, and virtually no rate-adaptation of the ERP was detectable. No significant differences were observed between ATP-only dogs and dogs subjected to atrial tachycardia in the presence of vitamin C (panel A), vitamins C and E (panel B) or sustained-release vitamin C (panel C). Dogs subjected to atrial tachycardia in the presence of simvastatin showed ERP values that were significantly greater than those subjected to ATP without drug intervention (panel D).

In non-paced control dogs, AF always terminated spontaneously within 5 minutes. AF requiring cardioversion for termination was induced following ATP in 57% of ATP-only dogs, 33% of vitamin C-treated dogs, 50% of combined vitamin C and E-treated dogs and 40% of sustained-release vitamin C-treated dogs. No sustained AF requiring cardioversion occurred in atrial tachypaced dogs treated with simvastatin. Figure 5 summarizes differences in mean AF duration and atrial vulnerability at open-chest study among the different groups of dogs. Non-paced control dogs had mean AF durations averaging 42 seconds (panel A), and ATP increased mean AF duration at open-chest study to over 1000 seconds. Dogs subjected to ATP in the presence of each of the antioxidant vitamin regimens had mean AF durations greater than 500 seconds and not significantly different from ATP-only. Dogs subjected to ATP in the presence of simvastatin had substantial attenuation of the AF maintenance-promoting effect of atrial tachycardia, with a mean AF duration (-40 seconds) equivalent to that of non-paced controls. AF vulnerability changes are shown in panel B. AF was induced by single extrastimuli at a mean of over 50% of atrial sites in ATP-only dogs, significantly greater than the less than 15% of sites at which AF could be induced in non-paced controls. In dogs subjected to ATP during therapy with antioxidant vitamins, AF was induced at an average of >50% of sites in each group. In simvastatin-treated dogs exposed to ATP, atrial vulnerability was significantly reduced compared to ATP-only, to an average of ∼20%.

Figure 6 shows values of atrial ERP in different atrial regions. ERP decreases caused by ATP were regionally variable, as previously described,¹⁶ with the largest changes occurring in RA inferior wall, posterior wall and appendage, as well as LA appendage. There were no significant differences between ERP values in ATP-only dogs and dogs in each of the antioxidant vitamin groups (panels A-C). Simvastatin significantly attenuated ATP effects on ERP in RA appendage, posterior wall and inferior wall. LA ERP reductions induced byATP were not significantly altered by simvastatin therapy.

**Changes in L-Type Ca²⁺-Channel** α**-Subunit Protein Expression**

Reductions in L-type Ca²⁺-current,¹⁷ apparently due to transcriptional downregulation of the α1c pore-forming Ca²⁺-channel subunit, Caᵥ1.2,¹⁸⁻²⁰ are important in mediating electrophysiological changes caused by atrial tachycardia remodeling. The expression of Caᵥ1.2 protein in RA and LA appendage was therefore quantified with the use of Western blot techniques in non-paced dogs and dogs subjected to ATP during treatment with simvastatin, vitamin C and vitamins C and E. A clear signal was obtained at 207 kDa, corresponding to the expected molecular mass of Caᵥ1.2 protein (Figure 7A). ATP alone significantly reduced Caᵥ1.2 protein expression in both RA (Figure 7B) and LA (Figure 7C) tissue samples. Neither vitamin C alone nor vitamins C plus E altered the tachypacing-induced Caᵥ1.2 changes. In contrast, simvastatin significantly attenuated Caᵥ1.2 downregulation.

### Discussion

### Main Findings

Simvastatin was found to prevent AF promotion by 1 week of ATP in dogs. This action was associated with significant attenuation of RA ERP abbreviation and of atrial tachycardia-induced effects on Caᵥ1.2 protein expression. These actions were not shared by the antioxidant vitamin C nor by vitamins C and E in combination.

### Comparison with Previous Studies of Drug Effects on Atrial Tachycardia-Induced

### Remodeling

Although several articles have demonstrated beneficial effects of L-type Ca channel blockers on short-term atrial tachycardia-induced remodeling,²¹⁻²⁴ they appear to be ineffective against longer-term (>24-hour) remodeling.^{8,25} A variety of other compounds, including Na⁺, H⁺-exchange blockers and angiotensin converting-enzyme inhibitors, have also been found effective in short-term^{25,26} but not longer-term²⁸ AF. Carnes et al¹⁰ demonstrated effectiveness of vitamin C at doses equivalent to those in the present study in attenuating ERP changes caused by 48-hour atrial tachycardia in the dog (changes in arrhythmia promotion were not reported). Here, the effectiveness of vitamin C, alone or in combination with vitamin E, in preventing ERP or AF-promoting effects of 7-day atrial tachycardia was not observed.

The T-type Ca²⁺-channel blocker mibefradil^{7,8} and the broad-spectrum antiarrhythmic amiodarone⁹ do prevent the effects of 1-week atrial tachycardia in the dog. However, mibefradil has been removed from the market because of adverse drug interactions and amiodarone's value is limited by a range of potentially-serious adverse effects. The present study is believed to be the first to demonstrate the effectiveness of a HMG-CoA reductase (here, simvastatin) in atrial-tachycardia remodeling and AF promotion.

### Potential Underlying Mechanisms

Statins act as antioxidants by inhibiting superoxide production,²⁹ as well as by increasing nitric oxide bioavailability.^{30,31} Simvastatin increases catalase and glutathione peroxidase activity.³² Thus, without wishing to be bound by any particular hypothesis, it would appear that simvastatin's efficacy is due to an antagonism of oxidant pathways involved in atrial tachycardia remodeling.¹⁰ The antioxidant properties of both vitamin C and E are well-recognized,^{33,34} however, the ability of exogenous vitamin C and E to increase the body's already substantial stores of these important endogenous antioxidants may be insufficient to significantly alter atrial antioxidant capacity. An alternative explanation lies in the anti-inflammatory properties of statins^{14,15} in the context of the potential role of inflammation in AF.^{12,13} Although CRP concentrations were measured in the dogs used in the experiments described above, no significant changes with ATP or simvastatin administration were observed.

### Limitations of the Study

Simvastatin was much more effective in preventing tachycardia-induced RA ERP changes than those in the LA (Figure 6D). The basis of this regionally-determined efficacy is unclear, particularly in view of the ability of simvastatin to prevent LA Caᵥ1.2 downregulation (Figure 7C). These observations point to a role for factors other than Caᵥ1.2 downregulation in contributing to atrial-tachycardia induced ERP changes, and may be related to the observation that nitric-oxide synthase downregulation with atrial-tachycardia remodeling is more significant in LA than in RA.³⁵

### Potential Clinical Implications

Atrial-tachycardia remodeling has significant clinical consequences, particularly for AF occurrence and maintenance, and inhibition of such remodeling may be an interesting novel approach to AF therapy.⁶ To date, the drugs shown to prevent atrial-tachycardia remodeling in dog models have either been unavailable clinically (mibefradil) or have a variety of other potent electrophysiological and extra-cardiac actions (amiodarone). The doses of simvastatin that were used in the study (2 mg/kg daily) are equal to those used in some experimental dog studies³⁶ and smaller than in others,³⁷ but are somewhat higher than those in common clinical use (about 0.1 to 1 mg/kg).

### Conclusion

Simvastatin prevents the AF-promoting actions of atrial tachycardia in dogs, and may open up interesting new approaches to preventing the arrhythmic consequences of atrial-tachycardia remodeling in man.

### List of References

1. Dresing TJ, and Schweikert RA, Atrial Fibrillation. The Cleveland Clinic, Department of Cardiovascular Medicine, May 30, 1992.[Internet reference: http://www.clevelandclinicmeded.com/diseasemanagement/cardiolog/atrialfi brillation/atrialfibrillation.htm]
2. Wijffels MC, Kirchhof CJ, Dorland R, et al. Atrial fibrillation begets atrial fibrillation. A study in awake chronically instrumented goats. Circulation. 1995;92:1954-1968.
3. Morillo CA, Klein GJ, Jones DL, et al. Chronic rapid atrial pacing. Structural, functional, and electrophysiological characteristics of a new model of sustained atrial fibrillation. Circulation. 1995;91:1588-1595.
4. Elvan A, Wylie K, Zipes DP. Pacing-induced chronic atrial fibrillation impairs sinus node function in dogs. Electrophysiological remodeling. Circulation. 1996;94:2953-2960.
5. Gaspo R, Bosch RF, Talajic M, et al. Functional mechanisms underlying tachycardia-induced sustained atrial fibrillation in a chronic dog model. Circulation. 1997;96:4027-4035.
6. Nattel S. Therapeutic implications of atrial fibrillation mechanisms: can mechanistic insights be used to improve AF management? Cardiovasc Res. 2002;54:347-360.
7. Fareh S, Bénardeau A, Thibault B, et al. The T-type Ca2+ channel blocker mibefradil prevents the development of a substrate for atrial fibrillation by tachycardia-induced atrial remodeling in dogs. Circulation. 1999; 100:2191-2197.
8. Fareh S, Bénardeau A, Nattel S. Differential efficacy of L-and T-type calcium channel blockers in preventing tachycardia-induced atrial remodeling in dogs. Cardiovasc Res. 2001;49:762-770.
9. Shinagawa K, Shiroshita-Takeshita A, Schram G, et al. Effects of antiarrhythmic drugs on fibrillation in the remodeled atrium. Insight into the mechanisms of the superior efficacy of amiodarone. Circulation. 2003; 107:1440-1446.
10. Mihm MJ, Yu F, Carnes CA, et al. Impaired myofibrillar energetics and oxidative injury during human atrial fibrillation. Circulation. 2001;104:174-180.
11. Carnes CA, Chung MK, Nakayama T, et al. Ascorbate attenuates atrial pacing-induced peroxynitrite formation and electrical remodeling and decreases the incidence of postoperative atrial fibrillation. Circ Res. 2001;89:e32-e38.
12. Chung MK, Martin DO, Sprecher D, et al. C-reactive protein elevation in patients with atrial arrhythmias: inflammatory mechanisms and persistence of atrial fibrillation. Circulation. 2001;104:2886-2891.
13. Aviles RJ, Martin DO, Apperson-Hansen C, et al. Inflammation as a risk factor for atrial fibrillation. Circulation. 2003;108:3006-3010.
14. Weitz-Schmidt G. Statins as anti-inflammatory agents. Trends Pharmacol Sci. 2002;23:482-486.
15. Shishehbor MH, Brennan ML, Aviles RJ, et al. Statins promote potent systemic antioxidant effects through specific inflammatory pathways. Circulation. 2003;108:426-431.
16. Fareh S, Villemaire C, Nattel S. Importance of refractoriness heterogeneity in the enhanced vulnerability to atrial fibrillation induction caused by tachycardia-induced atrial electrical remodeling. Circulation. 1998;98:2202-2209.
17. Yue L, Feng J, Gaspo R, et al. Ionic remodeling underlying action potential changes in a canine model of atrial fibrillation. Circ Res. 1997;81:512-525.
18. Yue L, Melnyk P, Gaspo R, et al. Molecular mechanisms underlying ionic remodeling in a dog model of atrial fibrillation. Circ Res. 1999;84:776-784.
19. Brundel BJ, Van Gelder IC, Henning RH, et al. Ion channel remodeling is related to intraoperative atrial effective refractory periods in patients with paroxysmal and persistent atrial fibrillation. Circulation. 2001;103:684-690.
20. Brundel BJ, Van Gelder IC, Henning RH, et al. Gene expression of proteins influencing the calcium homeostasis in patients with persistent and paroxysmal atrial fibrillation. Cardiovasc Res. 1999;42:443-454.
21. Goette A, Honeycut C, Langberg JJ. Electrical remodeling in atrial fibrillation. Time course and mechanisms. Circulation. 1996;94:2968-2974.
22. Leistad B, Aksnes G; Verburg E, et al. Atrial contractile dysfunction after short-term atrial fibrillation is reduced by verapamil cut increased by BAY K8644. Circulation. 1996;93:1747-1754.
23. Daoud EG Knight BP, Weiss R, et al. Effects of verapamil and procainamide on atrial fibrillation-induced electrical remodeling in humans. Circulation. 1997;96:1542-1550.
24. Yu WC, Chen SA, Lee SH, et al. Tachycardia-induced changes of atrial refractory period in humans: rate dependency and effects of antiarrhythmic drugs. Circulation. 1998;97:2331-2337.
25. Lee SH, Yu WC, Cheng JJ, et al. Effect of verapamil on long-term tachycardia-induced atrial electrical remodeling. Circulation. 2000;101:200-206.
26. Jayachandran JV, Zipes DP, Weksler J, et al. Role of the Na+-H+ exchanger in short-term atrial electrophysiological remodeling. Circulation. 2000;101:1861-1866.
27. Nakashima H, Kumagai K, Urata H, et al. Angiotensin II antagonist prevents electrical remodeling in atrial fibrillation. Circulation. 2000;101:1861-1866.
28. Shinagawa K, Mitamura H, Ogawa S, et al. Effects of inhibiting Na+/H+-exchange or angiotensin converting enzyme on atrial tachycardia-induced remodeling. Cardiovasc Res. 2002;54:438-446.
29. Takemoto M, Node K, Nakagami H, et al. Statins as antioxidant therapy for preventing cardiac myocyte hypertrophy. J Clin Invest. 2001;108:1429-1437.
30. Laufs U, La Fata V, Plutzky J, et al. Upregulation of endotherial nitric oxide synthase by HMG CoA reductase inhibitors. Circulation. 1998;97:1129-1135.
31. Kureishi Y, Luo Y, Shiojima I, et al. The HMA CoA reductase inhibitor simvastatin activates the protein kinase Akt and promotes angiogenesis in normocholesterolemic animals. Nat Med. 2000;6:1004-1010.
32. Luo JD, Zhang WW, Zhang GP, et al. Effects of simvastatin on activities of endogenous antioxidant enzyme and angiotensin-converting enzyme in rat myocardium with pressure-overloaded cardiac hypertrophy. Acta Pharmacol Sin. 2002;23:124-128.
33. Frei B, England L, Ames BN. Ascorbate is an outstanding antioxidant in human blood plasma. Proc Natl Acad Sci USA. 1989;86:6377-6381.
34. Takahashi M, Tsuchiya J, Niki E. Scavenging of radicals by vitamin E in the membrane as studied by spin labeling. J Am Chem Soc. 1989;111:6350-6353.
35. Cai H, Li Z, Goette A, et al. Downregulation of endocardial nitric oxide synthase expression and nitric oxide production in atrial fibrillation: potential mechanisms for atrial thrombosis and stroke. Circulation. 2002;106:2854-2858.
36. Satoh K, Yamato A, Nakai T, et al. Effects of 3-hydroxy-3-methylglutaryl coenzyme A reductase inhibitors on mitochondrial respiration in ischaemic dog hearts. Br J Pharmacol. 1995;116:1894-1898.
37. Mital S, Zhang X, Zhao G, et al. Simvastatin upregulates coronary vascular endothelial nitric oxide production in conscious dogs. Am J Physiol (Heart Circ Physiol). 2000;279:H2649-H2657.

## Claims

1. The use of a therapeutically effective amount of a HMG - CoA reductase inhibitor, namely a statin drug in the manufacture of a medicament for preventing or attenuating atrial fibrillation (AF) promotion by atrial tachycardia in a subject.

2. The use according to claim 1, wherein said statin drug is effective against longer-term atrial tachycardia remodelling.

3. The use according to claim 1 or 2, wherein said drug is simvastatin (Zocor®).

4. The use according to claim 1, 2 or 3, wherein said subject is human.

## Patentansprüche

1. Verwendung einer therapeutisch wirksamen Menge eines HMG-CoA-Reduktase-Inhibitors, namentlich eines Statin-Arzneimittels, bei der Herstellung eines Medikaments zum Vermeiden oder Abschwächen des Förderns von Vorhofflimmern (AF) durch Vorhoftachykardie bei einem Probanden.

2. Verwendung gemäß Anspruch 1, wobei das besagte Statin-Arzneimittel gegen längerfristiges Remodeling bei Vorhoftachykardie wirksam ist.

3. Verwendung gemäß Anspruch 1 oder 2, wobei es sich bei dem besagtem Arzneimittel um Simvastatin (Zocor^{®}) handelt.

4. Verwendung gemäß Anspruch 1, 2 oder 3, wobei es sich bei dem besagten Probanden um einen Menschen handelt.

## Revendications

1. Utilisation d'une quantité efficace au plan thérapeutique d'un inhibiteur de la HMG-CoA réductase, à savoir un médicament de type statine, dans la fabrication d'un médicament visant à empêcher ou à atténuer la promotion d'une fibrillation auriculaire (AF) par une tachycardie atriale chez un sujet.

2. Utilisation selon la revendication 1, dans laquelle ledit médicament de type statine est efficace contre un remodelage à plus long terme résultant d'une tachycardie atriale.

3. Utilisation selon la revendication 1 ou 2, dans laquelle ledit médicament est la simvastatine (Zocor®).

4. Utilisation selon la revendication 1, 2 ou 3, dans laquelle ledit sujet est un être humain.
